# EUROPEAN PATENT SPECIFICATION

(11) **EP 0 959 888 B1**
(45) Date of publication and mention of the grant of the patent: **05.09.2001**
(21) Application number: 97932969.5
(22) Date of filing: 08.08.1997
(51) Int. Cl.: A61K 31/47

(54) **USE OF THE CHELATING AGENT CLIOQUINOL FOR THE MANUFACTURE OF A PHARMACEUTICAL COMPOSITION FOR THE TREATMENT OF ALZHEIMER'S DISEASE**
VERWENDUNG VON DEM CHELATBILDNER CLIOQUINOL ZUR HERSTELLUNG EINES ARZNEIMITTELSZUR BEHANDLUNG VON ALZHEIMERSCHEN KRANKHEIT
EMPLOI DU CHELATEUR CLIOQUINOL POUR PRODUIRE UNE PREPARATION PHARMACEUTIQUE TRAITANT LA MALADIE D'ALZHEIMER

(30) Priority: 13.08.1996 GR 96010286
(43) Date of publication of application: 01.12.1999
(73) Proprietor: P.N. GEROLYMATOS S.A., 145 65 Kryoneri Attikis (GR)
(72) Inventor: GEROLYMATOS, Panayotis, Nikolas, GR-145 65 Kryoneri Attikis (GR)
(74) Representative: Rasmussen, Torben Ravn
(86) International application number: PCT/IB97/00983
(87) International publication number: WO 98/06403

(56) References cited:
- WO-A-93/10459
- WO-A-95/31199
- DE-A- 3 932 338
- GB-A- 951 992
- US-A- 3 178 343
- H. TJÄLVE: "THE ETIOLOGY OF SMON MAY INVOLVE AN INTERACTION BETWEEN CLIOQUINOL AND ENVIRONMENTAL METALS" MEDICAL HYPOTHESES, vol. 15, 1984, pages 293-299, XP002047206
- A.I. BUSH ET AL.: "MODULATION OF ABETA ADHESIVENESS AND SECRETASE SITE CLEAVAGE BY ZINC" THE JOURNAL OF BIOLOGICAL CHEMISTRY, vol. 269, 1994, pages 12152-12158, XP002047207 cited in the application
- J.E.F. REYNOLDS: "MARTINDALE, THE EXTRA PHARMACOPOEIA" 1993 , THE PHARMACEUTICAL PRESS , LONDON XP002047208 " Clioquinol " see page 511

## Description

The present invention relates to the use of a chelating agent for the manufacture of a pharmaceutical composition for the treatment of Alzheimer's disease.

Alzheimer's disease is a chronic neurodegenerative disease prevalent primarily among elderly people.

The clinical diagnosis of Alzheimer's disease is supported by the existence and accumulation of amyloid deposits in the brain. The amyloid is primarily found in the terminal zones of neurons as morphologically heterogeneous deposits, also known as senile plaque. The formation of senile plaque is related to the appearance of the symptoms and signs of the disease, including amnesia. After the formation of senile plaque, neurofibrillary tangles are produced in the neuronal bodies. The formation of neurofibrillary tangles is related to the worsening of amnesia and of the other symptoms of dementia.

A major component of the amyloid deposits is a polypeptide referred to herein as Aβ (Amyloid-beta). Aβ is normally a soluble component of the cerebrospinal fluid where it is found in concentrations of about 3-5 nM. Aβ may have 39 to 43 amino acids, typically 40 amino acids, in the matured form and is derived as a proteolytic cleavage product from a cell surface protein called the amyloid precursor protein (APP) (Kang et al. 1987). The normal function of Aβ is not known at present but might be to form cation-selective channels across cell membranes (Kawahara et al., 1997).

The precipitation of synthetic Aβ has been shown to be caused by several environmental factors including low pH, high salt: concentrations and the presence of metals, e.g. zinc, copper, and mercury (Bush et al., 1995). It has been reported that Aβ itself specifically and saturable binds zinc of a high affinity binding (K_{D} = 107 nM) at a molar ratio of 1:1 (zinc: Aβ) (Bush et al., 1994a). This binding takes place at physiological concentrations of zinc (Bush et al., 1994b).

There is a strong supposition that the removal of amyloid deposits from patients suffering from Alzheimer's disease will alleviate the symptoms of Alzheimer's disease. Therefore, several attempts have been made to prepare a drug for the removal of amyloid deposits.

WO 93/10459 discloses a method for the treatment of Alzheimer's disease by administrating a zinc binding agent. As preferred compounds phytic acid, desferrioxamine, sodium citrate, EDTA, 1,2-diethyl-3-hydroxypyridin-4-one, and 1-hydroxyethyl-3-hydroxy-2-methylpyridin-4-one are mentioned.

DE 39 32 338 discloses the use of a chelator, such as 8-hydroxy-quinoline, for the treatment of Alzheimer's disease.

US 5373021 discloses disulfiram and its salts and analogues in as far as these can penetrate the blood-brain-barrier. The disclosed compounds may be used to reduce neurological damages caused by i.a. Alzheimer's disease.

The hitherto known compounds suggested for the treatment of Alzheimer's disease have several drawbacks, which has prevented their widespread use. E.g., most of the compounds are unable to penetrate the blood-brain-barrier and thus can hardly reach the areas in which the amyloid is deposited. Disulfiram, which may penetrate the blood-brain-barrier, has the drawback that it also is an alcohol deterrent.

US-A-3178343 and GB-A-951992 disclose an animal feed composed of a premix and a feed formula to be blended before use. The premix comprises, among other ingredients, clioquinol and the feed formula comprises, as one of several components, vitamin B₁₂.

WO-A-93/10459 relates to a method for treating Alzheimer's disease by modulating divalent cation, trivalent cation and/or heparin interaction with APP.

DE-A-3932338 discloses chelation of aluminium ions in humans with an electron-donator-substituted quinoline or quinaldine oxidised at position 8. The chelation of aluminium ions may be used in the treatment of Alzheimer's disease.

The most extensively studied chelator is EDTA. However, the chelating effect of EDTA is relatively weak towards zinc and copper. Furthermore, EDTA cannot penetrate the blood-brain-barrier and is considered relatively toxic.

The object of the present invention is to provide a new use of a known pharmaceutical compound for the manufacture of a pharmaceutical composition for the treatment of Alzheimer's disease, said new compound having the ability to penetrate the blood-brain barrier, to effectively chelate heavy metals to prevent the aggregation of amyloid, and to redissolve amyloid deposits. This object is achieved by using clioquinol for such a new use.

The term "treatment of Alzheimer's disease" used herein refers to the prevention, control and therapy of Alzheimer's disease at any stage of its development.

Clioquinol has the chemical name 5-chloro-7-iodo-8-hydroxyquinoline and belongs to the group of hydroxy-quinolines. Clioquinol has a known use as topical anti-infective agent. Especially, clioquinol has been used for the treatment of amoebiasis and infectious diarrhoea. Clioquinol is only scarcely soluble in water within a pH range of 7-11. Outside this range suitable concentrations may be achieved.

The chelating ability of clioquinol is known for Fe, Co, Ni and Zn (Kidani et al., 1974 and Tateishi et al., 1973). Using mass spectrophotometry the coordination number for clioquinol in case of Co(II), Ni(II), Cu(II) and Zn(II) is 2, whereas the coordination number for Fe(III) is 3. Reportedly, injected preparations of clioquinol have crossed the blood-brain-barrier, leaving concentrations thereof in the brain in the order of 20*µ* l/ml when administrated at dosages of 10-20 mg/kg (Tateishi et al. 1975 and Tamura 1975). The concentration of clioquinol was also found to be high in such areas of the brain like the hippocampus which is immediately affected by Alzheimer's disease.

Using microautoradiograpic techniques, clioquinol has been shown in monkeys to form zinc chelates in the hippocampus. The Zn(II) chelates were mainly found in the terminal axodendritic boutons of the mossy fibres. Unconjugated clioquinol has an extremely rapid penetration when injected intravenously into the nervous system with practically no blood-brain-barrier (Shiraki, 1979).

Even though clioquinol had a known chelating and blood-brain-barrier penetrating ability it could not have been predicted that clioquinol also had the ability to redissolve zinc precipitated Aβ. According to the present invention the new use of clioquinol for the manufacture of a pharmaceutical composition for the treatment of Alzheimer's disease is based on this unexpected finding.

At present, it is believed that clioquinol and Aβ competitively chelate zinc and other heavy metals. Clioquinol is regarded as the strongest chelator and will, therefore, predominately capture the heavy metal ions. Thus, Aβ from precipitated zinc-Aβ will be resolubilized into the surrounding fluid because clioquinol will capture the zinc ions. The complex of clioquinol and zinc will penetrate the blood-brain-barrier and be cleared from the organism.

Since clioquinol is a relatively strong chelator, it may also chelate metal ions from enzymes or prosthetic groups. Therefore, it might be desirable to supplement trace metal ions or prosthetic groups to clioquinol treated patients, especially when establishing prolonged clioquinol treatments.

Vitamin B₁₂ contains cobalt. Previous case studies of subacute myelo-optic neuropathy (SMON) in Japanese patients who were administrated clioquinol at higher dosages than recommended and for long periods indicate a connection between prolonged clioquinol treatment and vitamin B₁₂ deficiency. In the present application the interaction between clioquinol and B₁₂ is proved through *in vitro* and *in vivo* studies. Therefore, it may be beneficial to administrate vitamin B₁₂ together with or subsequent to the administration of clioquinol in order to prevent B₁₂ deficiency.

Clioquinol may be administrated in any appropriate amount in any suitable galenic formulation and following any regime of administration.

Preferably, the amount of daily administration will be from 10 mg to 750 mg clioquinol depending on the condition of the patient. A typically daily dosage is 100 mg. Alternatively, from 10 mg to 250 mg, preferable 100 mg clioquinol, three times daily, may be administrated. A daily dosage of up to 750 mg for a period of two weeks is considered without any risk of neurotoxicity or other side effects.

For the prevention of the onset of the symptoms and signs of Alzheimer's disease, or for the delay of the symptoms and signs in the evolution of the disease, daily clioquinol dosages of 10 mg to 100 mg can be administrated for long periods, viz. up to ten years.

The actual administrated amount is to be decided by the supervising physician and may depend on multiple factors, such as, the age, condition, file history, etc. of the patient in question. The results presented herein suggest that a "window" for the administrated amount of clioquinol exists, within which the best dissolving results are obtained. Such a window may be determined by the physician upon conducting routine experiments.

If clioquinol is going to be administrated for a prolonged period, clioquinol is preferably administrated intermittently. In a first period clioquinol may be administrated e.g. for one to three weeks, followed by a wash-out period, which may provide for restoration of any unwanted side effects of clioquinol. The duration of the wash-out period may be one to four weeks. During the wash-out period it is preferred to administrate B₁₂ and other prosthetic groups and/or trace metals. After the wash-out period the first period may be repeated. The long-term intermittent therapy will provide not only for the resolubilisation of zinc-Aβ aggregates but also for the prophylactic inhibition of the formation of zinc-Aβ aggregates.

The intermittent administration of clioquinol will also reduce the toxicity potential of the drug, which means that the treatment may be extended throughout the evolution of the disease.

The pharmaceutical composition comprising clioquinol may be any suitable galenical formulation for enteral and parenteral administration aiming at delivering sufficient concentrations of clioquinol into the brain. It is preferred to administrate clioquinol as intravenous injections if it is desired to obtain high brain concentrations rapidly. As it is more convenient to administrate a pharmaceutical composition orally, this way of administration may be used if it is not of importance to rapidly obtain high brain concentrations. The pharmaceutical composition may also be administrated intradermally.

The pharmaceutical composition comprising clioquinol may comprise other active ingredients as well. Especially, the composition may contain trace metals or prosthetic groups such as vitamin B₁₂, and/or any other therapeutic agent that can be used concurrently for the treatment of Alzheimer's disease to improve or alleviate the symptoms and signs of Alzheimer's disease, or to delay the onset of any of the symptoms and signs of Alzheimer's disease. Alternatively, the other active ingredient(s) may be administrated as separate pharmaceutical composition(s) together with the pharmaceutical composition comprising clioquinol.

According to the invention, the new use of clioquinol comprises:
1) the treatment of patients already diagnosed as having Alzheimer's disease at any clinical stage,
2) the prevention of the disease in the patients with early or prodromal symptoms and signs, and/or
3) the delay of the onset or evolution or aggravation or deterioration of the symptoms or signs of Alzheimer's disease.

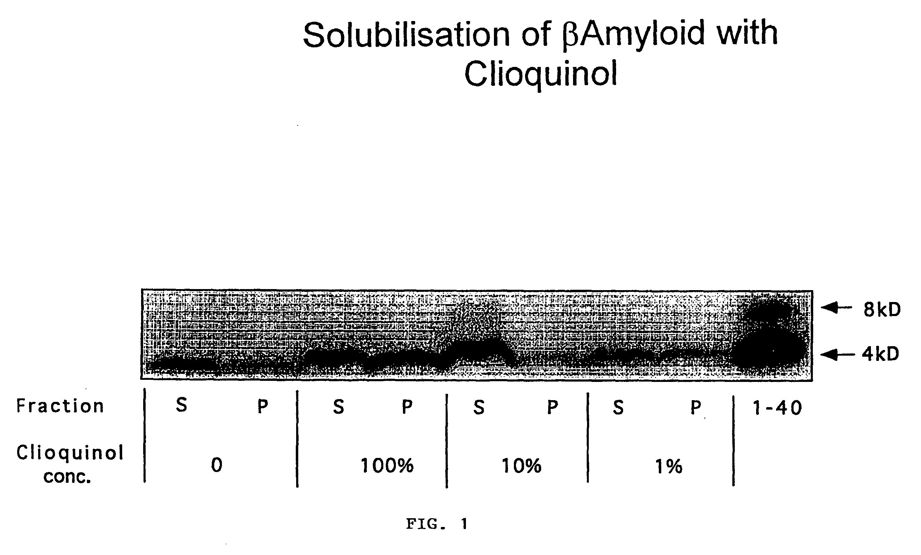

Brief description of the drawing: Fig. 1 depicts the solubilisation of β amyloid with clioquinol".

In the following the present invention will be illustrated by means of examples.

### EXAMPLES

### EXAMPLE 1

5.3 g of clioquinol was suspended with agitation in 200 ml of n-decane. The undissolved material was allowed to settle. Weighing the dried undissolved clioquinol after blowing off the decane indicated that only 2% of the clioquinol dissolved in the decane. 100 ml of the (light yellow) supernatant was agitated together with 100 ml of PBS (phosphate buffered saline) pH 7.4 and the phases allowed to separate. The PBS (lower phase) was collected and filtered to remove the residue which formed at the phase interface upon extraction with the organic solvent. Assuming that 2% of the clioquinol dissolved in the n-decane, and assuming that the partitioning coefficient is 1/1750 with PBS at 1:1 mixtures of decane/clioquinol, the concentration of clioquinol in the PBS is 800 nM.

Brain specimens were obtained for which a histophatological diagnosis of Alzheimer's disease was confirmed. Duplicate 0.5 g specimens of frontal lobe neocortex were homogenised in 3 ml of the clioquinol/ PBS solution at 100%, 10% and 1% of the final PBS/ clioquinol extract and PBS alone.

The homogenates were centrifuged at 150,000 xg for thirty minutes and the supernatants collected and held on ice (fraction "S"). The pellets were subjected to an identical homogenisation and centrifugation regime and the resulting supernatants again collected (fraction "P").

1 ml of each supernatant was treated with 200 *µ* l of ice cold 10% TCA (trichloroacetic acid) to precipitate total protein including A*β*. The resulting pellet was washed once with 100% ethanol and resuspended in 100 *µ*l of TBS (tris 20 mM, NaCl 150 mM pH 7.4).

7.5 *µ*l of sample (S or P) was boiled for 5 min with an equal volume of tris-tricine sample buffer containing 4% SDS (sodium dodesyl sulphate) and loaded onto a Novex pre-cast 10-20% tris-tricine gel followed by Western transfer onto nitrocellulose. Signal for Aβ was detected using mAb WO2 (raised against residues 5-16 of Aβ) and visualized using ECL (emitter coupled logic). The sensitivity of the detection system is 5-10 pg.

To validate the TCA precipitation for Aβ 1 *µ*g of synthetic Aβ 1-40 was added to 1 ml of PBS containing 10% BSA and the solution was treated as above. Signal for Aβ was detected in the precipitated pellet but not in the supernatant.

The result is indicated in fig. 1.

As it might be deduced from fig. 1, the clioquinol was effective in promoting the solubilisation of Aβ in the concentrations tested. Furthermore, the optimal concentration was found to be "10%", indicating that one of the aggregation forms of Aβ, presumable the dimer, is more soluble in PBS than others.

While data are shown only for one specimen in fig. 1, data for 19 other specimens all indicate the same tendency, viz. that clioquinol is effective in promoting the solubisation of Aβ.

### Example 2

In this example the chelating ability of clioquinol and EDTA is compared.

Samples of 10 ng synthetic Aβ were placed in microtitre wells and caused to aggregate by the addition of 25 *µ*M ZnCl. The aggregates were then transferred to a 0.2 *µ*m nylon membrane by filtration. The aggregates were washed with 200 *µ*l TBS alone, TBS containing 2 *µ*M EDTA, and TBS containing 2 *µ*M clioquinol. The membrane was fixed, probed with the anti-Aβ monoclonal antibody 6E10 and developed for exposure to ECL-film. The transmittance of the ECL-film was measured and the relative signal strength calculated based on 100% for TBS alone. The relative signal strength was 66% for EDTA and 49% for clioquinol.

The results indicate that clioquinol is a better chelator for zinc precipitated Aβ than EDTA.

### EXAMPLE 3

In this example the resolubilisating effect of clioquinol is demonstrated.

A 2.5 *µ*M. solution of Aβ in TBS at a pH of 7.4 was prepared. 95% of the Aβ was maintained in a soluble state. Addition of 30 *µ*M zinc resulted in precipitation of the soluble Aβ and only 43% was maintained in solution. The subsequent addition of 120 *µ*M clioquinol to the zinc precipitated Aβ resulted in an increase of soluble Aβ to 70%.

The results indicate that clioquinol is able to redissolve zinc precipitated Aβ.

### EXAMPLE 4

In this example the influence of clioquinol on vitamin B₁₂ is studied *in vitro* using NMR spectroscopy.

Since clioquinol is practically insoluble in water within the pH range of 7-11, the study was conducted at pH 13. Three tubes were prepared. The first contained 1 mg clioquinol in 0.5 ml, the second contained 1.4 mg cyanocobalamine (B₁₂) in 0.5 ml, and the third contained 0.5 mg clioquinol + 0.7 mg cyanocobalamine in 0.5 ml (mol ratio 3:1).

The ¹H NMR spectra were recorded in a DRX 400 MHz spectrophotometer at 20°C. Comparison of the third spectra with the first and the second shows that some of the resonances of cyanocobalamine are shifted and the same is observed for two of the resonances of clioquinol. The results suggest an interaction between clioquinol and cyanocobalamine.

### EXAMPLE 5

In this example the influence of clioquinol on vitamin B₁₂ is studied *in vivo*.

Six weeks old male mice were divided into two groups, a control group and a group which was pretreated with clioquinol for three days (50mg/kg/day). The mice were injected with [⁵⁷Co]-cyanocobalamine. 48 hours after the injection the animals were sacrificed, and the brain, liver and kidney were dissected and counted in a gamma-counter as thousand cpm/g tissue (wet weight) ± SEM. The radioactivities in each of the groups are stated in Table 1 below:

**Table 1**

| Group | Brain | Liver | Kidney |
|---|---|---|---|
| Control | 9.4 ± 0.9 | 97 ± 8 | 895 ± 207 |
| Clioquinol | 8.4 ± 1.5 | 85 ± 21 | 252 ± 61 |

A comparison of the results show that there were no significant changes in the amount of radioactive accumulation in the brain and the liver. A reduction in the amount of vitamin B₁₂ trapped in the kidneys was apparent. It may be concluded that clioquinol has an influence on the concentration of B₁₂ in some of the mice organs.

### REFERENCES

Kang et al. (1987) Nature **325:** 733-736.

Kirschner et al. (1987) Proc. Natl. Acad. Sci. **267:** 6953-6957.

Kawahara M.; Arispe N.; Kuroda Y.; Rojas E. (1997) Biophysical Journal **73/1,** 67-75.

Bush, A.I., Moir, R.D., Rosenkranz, K.M., and Tanzi, R.E. (1995) Science **268:** 1921-1923.

Bush, A.I., Pettingell Jr., W.H., Paradis, M.D., and Tanzi, R.E. (1994a) J. Biol. Chem. **269:** 12152-12158.

Bush, A.I., Pettingell Jr., W.H., Multhaup, G., Paradis, M.D., Vonsattel, J.P., Gusella, J.F., Beyreuther, K., Masters, C.L., and Tanzi, R.E. (1994b) Science **265:** 1464-1467.

Esler, W.P., Stimson, E.R., Jennings, J.M., Ghilardi, J.R., Mantyh, P.W., and Maggio, J.E. (1996) Neurochem. **66:** 723-732.

Kidani Y et al., (1974) Jap. Analyst **23**:1375-1378

Tateishi J etl., (1973) Psychiat. Neurol. Jap. **75:** 187-196.

Shirilogi H., et al., (1979) Handbook of Clinical Neurology, North Holland Publishing Company, 141-198.

Tamura Z, (1975), Jap. J. med. Sci.Biol. Suppl **28:** 69-77.

## Claims

1. Use of clioquinol for the manufacture of a pharmaceutical composition for the treatment of Alzheimer's disease.

2. Use according to claim 1, wherein the clioquinol is to be administrated in an amount of 10 to 250 mg one to three times daily.

3. Use according to claim 1 or 2, wherein trace metals and/or prosthetic groups are to be administrated together with or subsequent to the administration of clioquinol.

4. Use according to claim 3, wherein the prosthetic group is vitamin B₁₂.

5. Use according to any of the preceding claims, wherein the pharmaceutical composition comprising clioquinol is to be administrated intermittently.

6. Use according to claim 5, wherein the pharmaceutical composition comprising clioquinol is to be administrated for one to three weeks followed by a wash-out period of one to four weeks.

7. Use according to claim 6, wherein trace metals and/or prosthetic groups are to be administrated during the wash out period.

8. Use according to claim 7, wherein the prosthetic group is vitamin B₁₂.

9. Use according to any of the preceding claims, wherein treatment is to be extended up to ten years.

10. Use according to any of the preceding claims, wherein the pharmaceutical composition comprising clioquinol is formulated for oral administration.

11. Use according to any of the claims 1 to 9, wherein the pharmaceutical composition is formulated for parenteral administration.

12. Use according to any of the claims 1 to 9, wherein the pharmaceutical composition is formulated for intradermal administration.

## Patentansprüche

1. Verwendung von Clioquinol zur Herstellung eines Arzneimittels für die Behandlung von Morbus Alzheimer.

2. Verwendung von Clioquinol nach Anspruch 1, wobei die Verabreichung in einer Menge von 10 bis 250 mg eins bis dreimal täglich erfolgt.

3. Verwendung von Clioquinol nach Anspruch 1 oder 2 bei gleichzeitiger bzw. nachträglicher Verabreichung von Spurmetallen und/oder prosthetischen Gruppen.

4. Verwendung von Clioquinol nach Anspruch 3, wobei die prosthetische Gruppe Vitamin B₁₂ ist.

5. Verwendung von Clioquinol nach einem der obigen Ansprüche bei intermittierender Verabreichung des clioquinolhaltigen Arzneimittels.

6. Verwendung von Clioquinol nach Anspruch 5, wobei die Verabreichung des clioquinolhaltigen Arzneimittels eine bis drei Wochen dauert und nachher ein eine bis vier Wochen langes Washout erfolgt.

7. Verwendung von Clioquinol nach Anspruch 6, wobei während der Washout-Phase Spurmetalle und/oder prosthetische Gruppen zu verabreichen sind.

8. Verwendung von Clioquinol nach Anspruch 7, wobei die prostetische Gruppe Vitamin B₁₂ ist.

9. Verwendung von Clioquinol nach einem der obigen Ansprüche, wobei die Behandlung bis auf zehn Jahre verlängert werden muss.

10. Verwendung von Clioquinol nach einem der obigen Ansprüche, wobei das clioquinolhaltige Arzneimittel für orale Verabreichung vorgesehen ist.

11. Verwendung von Clioquinol nach einem der Ansprüche 1 bis 9, wobei das Arzneimittel für intradermale Verabreichung vorgesehen ist.

12. Verwendung von Clioquinol nach einem der Ansprüche 1 bis 9, wobei das Arzneimittel für intradermale Verabreichung vorgesehen ist.

## Revendications

1. Utilisation du clioquinol pour produire une composition pharmaceutique traitant la maladie d'Alzheimer.

2. Utilisation selon la revendication 1, où le clioquinol est administré en quantités allant de 10 à 250 mg une à quatre fois par jour.

3. Utilisation selon la revendication 1 ou 2, où des métaux traceurs et/ou des groupes prosthétiques sont administrés en même temps que, ou après l'administration du clioquinol.

4. Utilisation selon la revendication 3, où le groupe prosthétique est la vitamine B₁₂.

5. Utilisation selon l'une quelconque des revendications précédentes, où la composition pharmaceutique comprenant du clioquinol est administrée par intervalles.

6. Utilisation selon la revendication 5, où la composition pharmaceutique comprenant du clioquinol est administrée pour une à trois semaines suivies d'une période de rinçage d'une à quatre semaines.

7. Utilisation selon la revendication 6, où des métaux traceurs et/ou des groupes prosthétiques sont administrés lors de la période de rinçage.

8. Utilisation selon la revendication 7, où le groupe prosthétique est la vitamine B₁₂.

9. Utilisation selon l'une quelconque des revendications précédentes, où le traitement est prolongé jusqu'à dix ans.

10. Utilisation selon l'une quelconque des revendications précédentes, où la composition pharmaceutique comprenant du clioquinol est formulée pour une administration orale.

11. Utilisation selon l'une quelconque des revendications 1-9, où la composition pharmaceutique est formulée pour une administration parentérale.

12. Utilisation selon l'une quelconque des revendications 1-9, où la composition pharmaceutique est formulée pour une administration intradermique.
